# EUROPEAN PATENT SPECIFICATION

(11) **EP 3 515 409 B1**
(45) Date of publication and mention of the grant of the patent: **28.10.2020**
(21) Application number: 17786829.6
(22) Date of filing: 26.09.2017
(51) Int. Cl.: A61K 9/00, A61K 47/36, A61K 9/70, A61K 31/045, A61K 31/728, A61K 47/56, A61K 47/61

(54) **COMPLEX AND COMPOSITIONS FOR THE TREATMENT OF OPHTHALMIC AND DERMATOLOGICAL DISEASES**
KOMPLEX UND ZUSAMMENSETZUNGEN ZUR BEHANDLUNG VON AUGEN- UND HAUTERKRANKUNGEN
COMPLEXE AND COMPOSITIONS POUR TRAITER LES MALADIES OPHTHALMOLOGIQUES ET DERMATOLOGIQUES

(30) Priority: 26.09.2016 IT 201600096125
(43) Date of publication of application: 31.07.2019
(73) Proprietor: NTC S.r.l., 20124 Milano (IT)
(72) Inventor: BUSETTI, Cesare, 20149 Milano (IT); ZELASCHI, Daniela, 20148 Milano (IT)
(74) Representative: Coppo, Alessandro
(86) International application number: PCT/EP2017/074337
(87) International publication number: WO 2018/055194

(56) References cited:
- EP-A1- 1 444 984
- WO-A1-2009/018060
- WO-A1-2015/007773
- US-A- 5 529 987
- ANNY M.S. CHENG ET AL: "Recent advances on ocular Demodex infestation :", CURRENT OPINION IN OPHTHALMOLOGY, vol. 26, no. 4, 1 July 2015 (2015-07-01), pages 295-300, XP055371885, US ISSN: 1040-8738, DOI: 10.1097/ICU.0000000000000168
- Anonymous: "T4O Pads (Terpinen-4-ol) for Ocular Demodex : Rosacea Support Group", , 3 August 2010 (2010-08-03), XP055371927, Retrieved from the Internet: URL:https://rosacea-support.org/t4o-pads-t erpinen-4-ol-for-ocular-demodex.html [retrieved on 2017-05-11]
- SEAN TIGHE ET AL: "Terpinen-4-ol is the Most Active Ingredient of Tea Tree Oil to Kill Demodex Mites", TRANSLATIONAL VISION SCIENCE & TECHNOLOGY, vol. 2, no. 7, 1 November 2013 (2013-11-01), page 2, XP055372006, DOI: 10.1167/tvst.2.7.2
- Anonymous: "Blephapad", , 5 September 2016 (2016-09-05), XP055372026, Retrieved from the Internet: URL:http://www.italianinnovation.net/uploa d/img/75db686b-c6fb-484c-a8e2-35f720f78b92 .pdf [retrieved on 2017-05-11]

## Description

The present invention concerns a complex and compositions for the treatment of ophthalmic and dermatological diseases.

Specifically, the present invention relates to a complex based on cross-linked hyaluronic acid for the treatment or the prevention of ophthalmic diseases or dermatological diseases, caused also by mite infestations.

The compositions of the invention find application in the cosmetic, dermatological, and ophthalmic fields.

### STATE OF THE ART

Blepharitis is an inflammatory process of the free edge of the eyelid that can extend to the skin, conjunctiva, follicles, ciliary glands, and Meibomian glands. Blepharitis may have subacute or chronic evolution, with resistance to therapeutic treatments, and tendency to recurrences. The main symptoms of blepharitis are heartburn, itching, tearing, feeling of heat or weight, photophobia, and blepharospasm.

Desquamation, in addition to eye irritation and slight blemish, triggers a vicious circle that feeds blepharitis. The presence of external agents on the eyelid margin creates a physical barrier to the physiological drainage of glandular ducts, obstructing them, and creating environmental conditions conducive to the formation of bacterial colonies.

The etiology of blepharitis is manifold. Blepharitis may be caused by constitutional, allergic, endocrine, environmental factors, such as dust and smoke, by avitaminosis, dyspepsia, food auto-poisoning, diabetes, or by microorganisms, usually staphylococci or mites.

A common concause of blepharitis is represented by an infestation of the periocular and pilosebaceous area by *Demodex folliculorum,* an ectoparasite belonging to the class of Arachnida, subclass Acarina.

This mite feeds on fat cell secretions, and by infesting the eyelash follicles it may also cause their fall.

Although there are many species of this mite, only *Demodex brevis* and *Demodex folliculorum* can infest human beings.

*Demodex* infestation is rather widespread, and it is usually contracted as a result of direct contact with the skin of an infested individual, or from dust containing eggs thereof.

A study by Junemann A., titled *Demodex folliculorum* in chronic blepharitis, revealed that infestation by *Demodex folliculorum* is present in about 25% of subjects 20 years old, 30% of 50 years old, and 100% of patients over 90 years old.

It has also been found that, at intervals of about ten lashes, one or more *Demodex* mites can nest even in healthy asymptomatic patients.

*Demodex folliculorum* generally forms colonies, within hair follicles and on the lashes. The presence of *D*. *folliculorum* is often associated with anterior blepharitis.

The main symptomatology attributable to *Demodex folliculorum* infestation includes eyelid redness, foreign body sensation, eyelid and eyebrow itching, madarosis, and burning sensation.

It has also been observed that *D. folliculorum* infestation causes follicular hyperplasia, resulting in increased keratinization near the eyelid base. In addition, *Demodex* infested eyelashes are often fragile and easily lost.

It has also been found that infestation by another mite species, such as *Demodex brevis,* which generally lives in the sebaceous glands of skin and eyelids, may contribute to the development of blepharitis.

The prevention of blepharitis is carried out through careful eyelid hygiene, which involves the removal of external agents, and maquillage, and secretions, and sebum from eyelashes and periocular area

However, in many cases blepharitis is not completely eradicable. In addition, if not treated properly, it can cause complications such as the formation of sty, chalazion, loss of eyelashes, and recurrent conjunctivitis.

The treatment of blepharitis, particularly infectious and ulcerative forms, is based on the application of topical products, such as ointments, creams, and unguents containing antibiotics, possibly in combination with corticosteroids.

The treatments currently available on the market have not been fully effective, since they cannot completely eradicate the infestation and, in some cases, lead to the formation of bacterial colonies that develop resistance to the antibiotics applied.

It is also known from US Patent N. 8,455,015B2A a composition for the treatment of *Demodex* infestations, containing as an active ingredient Tea Tree Oil (TTO), an essential oil derived from *Melaleuca alternifolia,* a plant originating from eastern Australia, belonging to the Myrtaceae family.

TTO consists of a mixture of about one hundred substances including terpene hydrocarbons, mainly monoterpenes, sesquiterpenes, and alcohols thereof, obtained by steam distillation of *Melaleuca alternifolia* leaves.

Of the components present in the plant extract, seven were found to have an anti-inflammatory or broad spectrum antibacterial activity. S. Tighe et al. "Terpinen-4-ol is the Most Active Ingredient of Tea Tree Oil to Kill Demodex Mites",Trans. Vision Science & Technology 2013, vol. 2, no. 7, page 2 discloses that terpinen-4-ol is the most active ingredient in TTO to exert antibacterial and antifungal effects.

WO 2009/018060 A1 discloses an aqueous ophthalmic composition comprising a natural polymer selected from hyaluronic acid and terpene compound such as 1-terpinen-4-ol. EP 1 444 984 A1 discloses a topical pharmaceutical composition comprising hyaluronic acid and Melaleuca alternifolia extracts. "Blephapad" (http://www.italianinnovation.net/upload/ img/75db686b-c6fb-484c-a8e2-35f720f78b92.pdf, 05-09-2016) discloses pads comprising hyaluronic acid and 1-terpinen-4-ol (as a complex) for the treatment of eyelid disorders.

The publication by Brand C, Grimbaldeston MA, Gamble JR, Drew J, Finlay-Jones JJ, Hart PH. "Tea tree oil reduces the swelling associated with the efferent phase of a contact hypersensitivity response"; Inflamm Res 2002; 51: 236-44 reports the anti-inflammatory activity of TTO.

The publications by Dryden MS, Dailly S, Crouch M. "A randomized, controlled trial of tea tree topical preparations versus a standard topical regimen for the clearance of MRSA colonization"; J Hosp Infect 2004; 56:283-6, and by Kwiecinski J, Eick S, Wojcik K. Effects of tea tree (Melaleuca alternifolia) oil on Staphylococcus aureus in biofilms and stationary growth phase; Int J Antimicrob Agents 2009;33:343-7 report the antibacterial action of TTO against microorganisms, including germs commonly isolated from the oral cavity of oncologic patients, such as Staphylococcus aureus, including MRSA (Methicillin-resistant Staphylococcus aureus).

The publication by Jobbins J, Bagg J, Parsons K, Finlay I, Addy M, R.G. Newcombe "Oral carriage of yeasts, coliforms and staphylococci in patients with advanced malignant disease" in J. Oral Pathol Med 1992;2; 21 (7): 305-8 reports the action of TTO against infections by coliform bacteria.

The use of TTO in the treatment of Herpes simplex virus, whose reactivations constitute a further serious clinical problem for terminal patients, is also known from Carson CF, Ashton L, Dry L, Smith DW, Riley TV in "Melaleuca alternifolia (tea tree) oil gel (6%) for the treatment of recurrent herpes labialis," J Antimicrob Chemother 2001; 48:450-1.

TTO, although considered a safe product for external applications, can cause allergic reactions, as documented by Veien NK, Rosner K, Skovgaard GL (2004) "Is tea tree oil an important contact allergen?" Contact Dermatitis 50: 378-379, or irritation and skin rashes, burning, itching, or contact dermatitis.

It has also been observed that TTO, in pure or diluted form, is irritant to mucous membranes, especially the eye mucous membrane, as documented in Southwell IA, Freeman S, Rubel D (1997), "Skin irritancy of tea tree oil", J Essent Oil Res 9: 47-52).

Therefore, TTO has a number of limitations for local application in sensitive skin areas, such as the periocular area.

Moreover, the external use of TTO active components is limited because of their strong organoleptic component.

In the International application WO 2015/007773 A1 to I.R.A. S.r.l., the inventors Dr. Citernesi et al. disclose the preparation of a biocompatible crosslinked biopolymer comprising hyaluronic acid cross-linked with urea and their applications. Currently, therefore, the need is felt to have products and compositions for ophthalmic and/or dermatological use having a disinfestation effect against mites and other microorganisms infesting the skin.

One of the aims of the invention consists in proving a product or composition which is applicable in the prevention and treatment of blepharitis and/or mite infestation, in particular *Demodex.*

Another aim consists in providing an ophthalmic composition for the treatment of infections of bacterial origin and/or mite infestations in the periocular area, whose use involves reduced risk of triggering local allergic reactions, or inflammation, or irritation of the treated skin areas.

A further aim of the invention consists in providing a topical or ophthalmic composition for the treatment of blepharitis or mite infections, that does not have an intense, persistent, and annoying smell perceived by the subject in need of treatment.

### SUMMARY

The inventors have found that the combination of a selected component of TTO, provided with antibacterial and acaricidal activity, with a skin-compatible cross-linked polymer, results in effective treatment of blepharitis and/or mite infestation, in particular by *Demodex,* in the substantially absence of local adverse reactions.

It has also been surprisingly found that by complexing terpinen-4-ol, an active component of TTO, with a cross-linked polymer based on hyaluronic acid, the organoleptic appearance of the acaricidal component greatly improves.

According to a first aspect, the present invention therefore provides a topical composition, especially for the prevention and/or treatment of ophthalmic and/or dermatological diseases, comprising
- a component with acaricidal and/or biocidal activity
- a physiologically acceptable cross-linked polymer, and a dermatologically acceptable vehicle,
characterized in that the acaricidal component is terpinen-4-ol, and the crosslinked polymer is cross-linked hyaluronic acid.

Further embodiments of the composition of the invention are defined in the appended claims 2-5.

In accordance with certain aspects, in the composition of the invention the cross-linked hyaluronic acid and terpinen-4-ol form a complex.

A further object of the present invention is therefore a complex based on hyaluronic acid cross-linked with urea and terpinen-4-ol.

The complex according to this aspect of the invention therefore comprises two components:
a) terpinen-4-ol, and b) a cross-linked polymer based on hyaluronic acid cross-linked with a cross-linking agent.

A suitable cross-linking agent is selected from water-soluble carbodiimides, divinyl sulfone, 1,4-butanediol diglycidyl ether, cross-linking agents containing multiple amino groups, and urea.

According to some preferred embodiments, the cross-linked polymer of the complex of the invention is hyaluronic acid cross-linked with urea.

Typically, the complex of the invention is a biocompatible and/or physiologically acceptable cross-linked macromolecular matrix

In accordance with another aspect, the present invention provides a composition for the prevention and/or treatment of blepharitis and/or skin infestations by mites, comprising terpinen-4-ol and a physiologically acceptable cross-linked polymer based on cross-linked hyaluronic acid for use in the treatment of blepharitis, mite infestations, acne, rosacea.

The composition of the invention finds specific application in the treatment of *Demodex* infestations and *Demodex*-induced blepharitis.

Typically, the composition of the invention is used for external use, specifically for application to the skin, for example in the periocular area and on all appendages of the eye.

### BRIEF DESCRIPTION OF THE FIGURES

The present invention will be described in detail below, and referring to the figures, in which:
Figure 1 shows a photographic reproduction of a 20x resolution microscope view of Dermatophagoides pteronyssinus on a Petri dish treated according to Example 5 of the description;
Figure 2 shows a further photographic reproduction of a 40x resolution microscope view of Dermatophagoides pteronyssinus on a Petri dish treated according to Example 5 of the description;
Figure 3 shows bar graphs related to comparative assessment tests of tolerability, and organoleptic characteristics in the use of towelettes soaked with Tea Tree Oil (TTO), Terpinen-4-ol (T4O), and Cross-linked Hyaluronic Acid/Terpinen-4-ol (Hy-Ter) of the invention.

### DETAILED DESCRIPTION OF THE INVENTION

In accordance with a first aspect of the invention, a topical composition is thus provided as defined in the appended Claim 1.

In accordance with another aspect, the invention relates to a complex of terpinen-4-ol with a hyaluronic acid polymer cross-linked with a physiologically or cosmetically acceptable cross-linking agent.

One of the advantages of the composition and complex of the invention lies in being substantially free of the pungent and persistent smell typical of the preparations containing Tea Tree Oil (TTO), or extracts thereof.

### Terpinen-4-ol of the Complex

One of the components of the composition and complex of the invention is terpinen-4-ol, a monoterpene alcohol with the IUPAC name of 1-isopropyl-4-methyl-3-cyclohexen-1-ol.

Terpinen-4-ol of the complex of the invention may be of synthetic or natural origin. For example, it may be obtained by extracting TTO obtained from tea tree leaves. According to some aspects of the invention, the Applicant has found that by complexing a selected monoterpene alcohol with a polymer based on cross-linked hyaluronic acid, a product with improved compatibility is obtained.

### Cross-linked Hyaluronic Acid of the Complex

Another component of the composition and complex of the invention is a polymer based on cross-linked hyaluronic acid.

Typically, the polymer based on hyaluronic acid can be cross-linked with a cross-linking agent selected from urea, water soluble carbodiimides, divinyl sulfone, 1,4-butanediol diglycidyl ether, or cross-linking agents containing multiple amino groups.

In accordance with a preferred embodiment, the cross-linked hyaluronic acid of the complex of the invention is cross-linked with urea.

### Embodiment with Hyaluronic Acid Cross-linked with Urea

In some embodiments, the polymeric component of the complex based on hyaluronic acid cross-linked with urea and terpinen-4-ol has a molecular weight ranging from 100,000 to 20,000,000 dalton, from 500,000 to 10,000,000 dalton, from 1,000,000 to 6,000,000 dalton, typically from 2,000,000 to 4,000,000 dalton, preferably from 1,500,000 and 1,800,000 daltons.

Typically, the hyaluronic acid of the cross-linked polymeric component of the complex is a polysaccharide, formed by alternating units of glucuronic acid and N-acetyl glucosamine, capable of retaining water and withstanding hydrostatic stresses.

Typically, the molecular weight of polymers described herein, such as hyaluronic acid is a weighted-average molecular weight (Mw) which may be determined preferably with SEC-MALLS technique, or multi-angle laser light scattering - size exclusion chromatography.

In some embodiments, in the complex of the invention the hyaluronic acid cross-linked with urea can be prepared separately by cross-linking hyaluronic acid, or a salt thereof, with urea, typically in an acidic environment. For example, hyaluronic acid, or salts thereof, and urea can be dissolved and/or combined in a liquid, for example water, in which both components are soluble. In some embodiments, hyaluronic acid in aqueous solution is present in a quantity ranging from 2 mg/ml to 120 mg/ml, from 5 to 25 mg/ml, typically from 7 to 20 mg/ml. In accordance with some embodiments, the urea in acidic aqueous solution is present in a concentration ranging from 1 to 100 mg/ml, from 3 to 50 mg/ml, typically from 5 to 25 mg/ml.

According to some embodiments, the weight ratio between hyaluronic acid, or a salt thereof, and urea in the aqueous solution or the cross-linking reaction mixture may range from 0.3 to 10, from 0.8 to 5, from 1 to 3.

In some embodiments, the obtained mixture has a pH ranging from 3.5 to 4. In some embodiments, an acid such as HCI is added to obtain the acidic pH of the solution.

Optionally, in the case where the mixture has a pH outside of 3.5-4, it is possible to add a pH correction agent, such as a solution of an acid, for example HCI, or a base, for example NaOH.

In some embodiments, to adjust the pH in the range of 3.5-4, a buffer such as for example acetic acid and sodium acetate may be added.

In some embodiments, the mixture is thermostated, for example at 35 (+/- 2) °C, for 20-24 hours, and then subjected to swelling to eliminate the urea excess present in solution.

In accordance with some embodiments, the pH of the obtained product ranges between 5.5 and 7.5. If the pH deviates from the range 5.5-7.5, it is possible to adjust it by adding a 0.2M NaOH solution, in one or more portions, until it falls within the desired range.

In certain embodiments, at the end of the cross-linking reaction, the obtained biocompatible cross-linked polymer is reduced into particles or homogenized through a screen having a pore size of, for example, between 5 and 150 microns, conveniently from 40 to 80 microns, to form a hydrogel.

### Embodiments of the Complex

In accordance with some embodiments, the complex has a terpinen-4-ol content ranging from 0.1 to 25% by weight, based on the total weight of the complex, from 1 to 20% by weight, from 3 to 18% by weight weight, from 5% to 15% by weight. Typically, the complex of the invention comprises an active component, represented by terpinen-4-ol, which is released from the component of the complex based on cross-linked hyaluronic acid.

In accordance with some embodiments, the complex of the invention is in the form of a hydrogel and preferably is a complex of terpinen-4-ol and hyaluronic acid crosslinked with urea (Examples 1 and 2).

In some embodiments, the complex of the invention is provided in a sterile form, suitable for medical or cosmetic applications.

### Method of Preparation of the Complex

In accordance with certain aspects, the present invention provides a method for preparing the complex of the invention according to one previously defined embodiment.

Typically, the method of preparation of the invention comprises the cross-linking of hyaluronic acid, or a salt thereof, with a cross-linking agent of the type previously described, preferably urea, in the presence of terpinen-4-ol, preferably in an acidic environment.

According to some embodiments, the method of preparing a hyaluronic acid cross-linked with urea-terpinen-4-ol complex comprises mixing a solution of terpinen-4-ol with a solution of hyaluronic acid, or a salt thereof, adding a solution of urea, and mixing, preferably maintaining the mixture at a pH of 3.5 to 4.

According to some embodiments, the product resulting from the previous step is brought to temperatures higher than room temperature, for example in the range of 30 to 40°C, for a period of time suitable to form the complex, for example 20-24 hours. In some embodiments, the resulting mixture is purified from the excess of urea until reaching a pH in the range from 5.2 to 7.2.

By way of example, the complex formed by hyaluronic acid cross-linked with urea and terpinen-4-ol can be prepared as follows.

Sodium hyaluronate is dissolved in an aqueous sodium chloride solution.

Separately, terpinen-4-ol is dissolved in a solution of Polysorbate 20 and pentylene glycol.

Separately, urea is dissolved in a 0.2M HCI solution.

The solution of terpinen-4-ol is added progressively to the solution of sodium hyaluronate maintained under continuous stirring. The stirring is continued until a homogeneous mixture is obtained.

The solution of urea is progressively added to the latter solution, maintained under vigorous stirring by means of a turboemulsifier.

The pH is measured and has to be within the range of 3.5-4.0; otherwise it is adjusted to this value.

The product is thermostated at 35°C ± 2°C for 20-24 hours, then purified by any excess of urea until the pH is within the range of 5.2-7.2.

### Definitions

Within the scope of the invention, the term "hyaluronic acid salt" is intended to include hyaluronic acid (free form) and any water-soluble salt of hyaluronic acid, such as sodium, potassium, or calcium salt.

Within the scope of the invention, the term "biopolymer" or "polymer" are equivalent, and both designate a physiologically, dermatologically, or cosmetically acceptable polymer, *i.e.* compatible with the epidermis of the human body.

The term "dermatologically acceptable" refers to vehicles or excipients that do not substantially cause any irritation, or inflammation, or skin damage when applied to it.

The term "ophthalmically acceptable" refers to non-detergent vehicles or excipients that do not substantially irritate or damage the surface of the eye and appendages thereof, such as eyelashes and eyebrows.

Within the scope of the present invention, the term "vehicle" refers to an excipient or diluent which has no pharmacological activity, and may be present in the composition of the invention, for example as a support element.

The term "complex" designates a product based on/comprising hyaluronic acid cross-linked with urea and terpinen-4-ol, wherein the hyaluronic acid cross-linked with urea is bound by secondary bonds and/or by weak molecular interactions to terpinen-4-ol.

### Compositions Containing the Complex

In accordance with one aspect, the present invention provides a composition comprising a complex based on cross-linked hyaluronic acid and terpinen-4-ol according to any one of the embodiments previously described, and a physiologically acceptable vehicle.

In accordance with a preferred embodiment, the composition of the invention comprises a complex based on hyaluronic acid cross-linked with urea and terpinen-4-ol.

The composition of the invention is in a form suitable for topical application.

Typically, the active component of the composition of the invention is represented by the cross-linked hyaluronic acid and terpinen-4-ol complex. As we have seen, terpinen-4-ol is released into the environment by the component of the cross-linked hyaluronic acid complex.

Typically, the physiologically acceptable vehicle of the composition is a dermatologically acceptable and/or ophthalmically acceptable vehicle.

In accordance with certain embodiments, the composition of the invention contains a dermatologically acceptable and/or ophthalmically acceptable excipient or vehicle, and a quantity of any embodiment of the complex in a quantity ranging from 0.1 to 20% by weight, from 0.5 to 15%, from 1 to 10% by weight.

Suitable formulations for topical application include solutions, suspensions, lotions, creams, gels, ointments, pastes, unguents, microemulsions, sprays, drops, foams, shampoos, roll-ons, substrates or devices to support the components of the composition, preferably wipes.

In some embodiments, the compositions of the invention may comprise excipients commonly used in the formulation of dermatological, cosmetic or pharmaceutical preparations for topical use, such as preservatives, bactericidal agents, stabilizers, emulsifiers, buffers, wetting agents, dyes, and other excipients commonly used in cosmetic/pharmaceutical preparation techniques.

In accordance with some embodiments of the invention, a device for the hygiene and/or disinfecting treatment of the periocular area comprising a substrate and a composition according to any one of the embodiments described above is provided.

In accordance with certain embodiments, the substrate is a fabric, for example hydrophilic cotton, or a nonwoven fabric, for example polyester, or a support based on polyurethane.

In some embodiments, the device is a wipe, a gauze, a towelette suitable for application to the ocular and/or periocular area.

In some embodiments, the device according to any one of the embodiments described above is wetted or soaked with a composition according to any one of the preceding embodiments.

In accordance with some embodiments, the device of the invention is wetted with an amount of composition from 0.001 to 0.1 g/cm².

Typically, the device wetted with the composition is packaged in a hermetically sealed container, such as an aluminum foil envelope or bag.

In some forms for applications of the device in the medical or ophthalmic field, the device is in a sterile form and/or vacuum packed. Sterilization may be carried out according to conventional procedures in pharmaceutical technology, for example by exposure to ethylene oxide atmosphere or gamma rays.

In accordance with some embodiments, the device is a medical device.

### Cosmetic and Medical Uses of Complex/Compositions

In the medical field, the complex of the invention and the compositions containing thereof find application in the treatment of diseases including mite infestations, in particular infestation by *Demodex,* by *Cheyletiella mite,* or by bacteria, as in the following non-exhaustive examples: blepharitis, acne, rosacea, acute or chronic conjunctivitis, conjunctivitis with allergic component. In general, the composition of the invention may be used for the treatment and control of infestations by any species of Demodex.

The composition of the invention finds specific use in treating *Demodex* infestations and/or blepharitis induced by *Demodex,* and in preventing or delaying recurrences.

In accordance with some embodiments, the complex and composition of the invention are used in the treatment of bacterial infections of the eye and/or skin, in particular by *Staphylococcus.* In particular, the composition of the invention finds application in the treatment of blepharitis and/or rosacea.

In the cosmetic field, the complex or composition of the invention find application in the detersion or hygiene of the periocular area, and eyelid and/or eyelashes. The composition of the invention may be applied or rubbed on eyelashes, eyelids, or periocular areas to clean or disinfect the contact area in order to eliminate contamination from external agents and/or mites.

In the embodiments of the device in the form of a gauze or wipe, it is appropriate to apply them on the eyelid surface, while keeping the eye closed, possibly by using circular movements that allow to deeply clean treated areas, and eliminate the impurities present on the epithelia, while maintaining a natural hydration of the skin, and underneath.

In the medical, ophthalmic field, the use of the device in the ocular or periocular area sanitizes the treated area, by reducing the bacterial charge present. In addition, the germicidal action is combined with a soothing action that reduces redness due to the action of environmental agents and/or microorganisms developed.

In accordance with some aspects, the device of the invention, in particular in the form of a gauze wetted with the composition, finds use in the medical field in the prevention and/or treatment of blepharitis, conjunctivitis, blepharoconjunctivitis, or infections of the lacrimal sac.

The present invention will now be described with reference to the following examples, that are provided for illustrative purposes only and should not be construed as limiting the scope of the present invention.

### EXAMPLE 1

Composition for topical use in the form of wipes containing the complex of the invention
Complex of cross-linked hyaluronic acid with urea and Terpinen-4-ol 5.0%
Aloe Barbadensis leaves, dry extract
PEG/PPG-20/15 Dimethicone
Ammonium glycyrrhizate
Caprylyl/Capryl glucoside
Sodium cocoyl amino acids
Ethylhexylglycerin
Phenylethyl alcohol
Sodium chloride
Sodium phosphate
Disodium phosphate
PPG-26 Buthet-26
PEG-40 hydrogenated castor oil
Water

### Method of Preparation

Raw materials, previously dispensed from the weighing room, are combined into a reactor and mixed until a homogeneous solution is obtained.

The wipes are produced by a special machine which provides to:
a) cut and form the gauze;
b) dose the previously prepared solution;
c) insert the soaked gauze into the coupled material of the sachet;
d) heat seal the sachet on 4 sides;
e) engrave a tear notch;
f) print variable data.

The sachets thus produced are packed in boxes together with the package leaflet.

### EXAMPLE 2

Composition for topical use in the form of a cream containing the complex of the invention.

### Composition of the Product:

**Phase A**

| | |
|---|---|
| Polyglyceryl-3-diisostearate | 4.50% |
| Polyglyceryl-6-dioleate | 3.00% |
| Glyceryl behenate | 2.00% |
| Vegetable squalene | 5.00% |
| Sweet almond oil | 2.00% |
| Vaseline oil | 10.00% |
| Tocopherol | 0.03% |

**Phase B**

| | |
|---|---|
| Demineralized water | 55.47% |
| Sodium chloride | 0.50% |
| Magnesium sulfate 7H₂O | 0.50% |
| Glycerin | 7.00% |

**Phase C**

| | |
|---|---|
| Demineralized water | 5.00% |
| Cross-linked hyaluronic acid/Terpinen-4-ol Complex | 5,00% |

### EXAMPLE 3

Method for preparing the composition of Example 2
Heat Phase A to about 80°C until all components are fully melted.
Heat Phase B to about 80°C.
While keeping Phase A under fast stirring with a turbine stirrer, slowly add Phase B.
Continue turbine stirring for about 15 minutes.
Cool under stirring to about 35°C, then add Phase C.
Complete cooling under gentle stirring.

### EXAMPLE 4

Components for preparing a composition of the disclosure wherein the hyaluronic acid sodium salt (component 2) is crosslinked with component 3 and component 4 is a crosslinking agent:

| | |
|---|---|
| 1. Terpinen-4-ol | 10.0% |
| 2.Hyaluronic acid sodium salt | 3.0% |
| 3.Urea | 4.0% |
| 4. Pentylene glycol | 5.0% |
| 5.Sodium chloride | 0.9% |
| 6.Polysorbate 20 | 10.0% |
| 7.Purified water | q.s.to 100% |

### EXAMPLE 5

### Activity of Wipes soaked with Composition of the Invention on Mites

This experiment was carried out to verify the acaricidal activity of the key component of *Melaleuca alternifolia* conjugated with hyaluronic acid (Complex of cross-linked hyaluronic acid-urea/Terpinen-4-ol) at different dosages (0.5%, 1%, and 2%) on *Dermatophagoides pteronyssinus* (DP) dust mites. Negative (neutral wipes) and positive (Cliradex, an American product containing a high dosage of Terpinen-4-ol) controls were also used in parallel, together with a commercial product containing phenylethyl alcohol, an organic component active against DP. *D. pteronyssinus* mites kept in laboratory for over 20 years without exposure to any known acaricide were used. Mites were bred in plastic containers (12.5 x 10.5 x 5.0 cm) at 25.0 ± 1.0 °C, at 75% relative humidity in a dark room, and fed with a proprietary diet.

Each wipe, formulated with different concentrations of Hyaluronic acid/Terpinen-4-ol Complex or alcohol, was placed on the bottom of a Petri plate (5 cm diameter, 1.2 cm high). A quantity of about 300 DPs were placed on the wipe in each Petri plate, which were then closed with the lid.

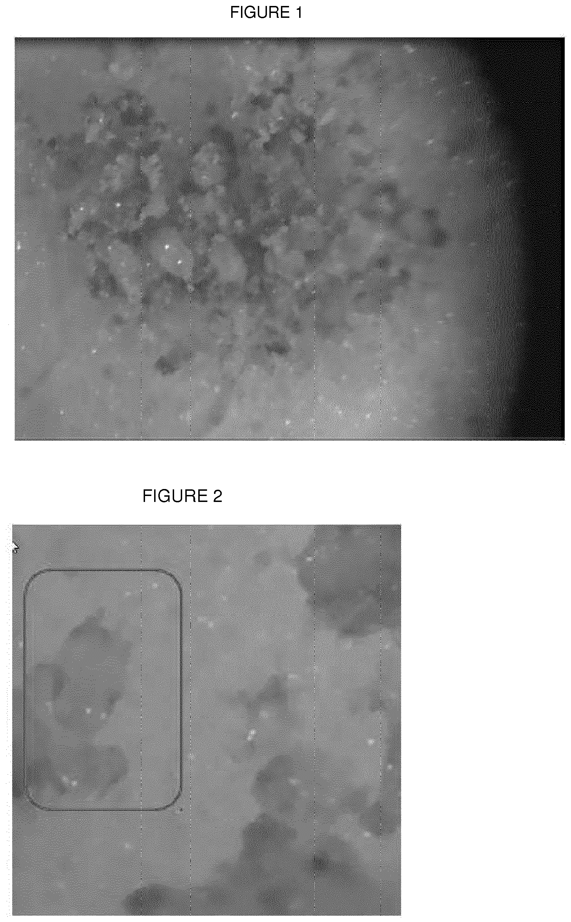

The movement of body and legs of the *Dermatophagoides pteronyssinus* (Figures 1 and 2) was continuously observed with a binocular microscope for up to 30 minutes.

"100% Mortality Time (MT)" was defined as the time by which all DP movements ceased.

The acaricidal effect of the Cross-linked hyaluronic acid/Terpinen-4-ol Complex is dose dependent, as shown in Table 1.

**Table 1:**

| Mortality of *Dermatophagoides pteronyssinus* (%) versus Cross-linked hyaluronic acid/Terpinen-4-ol Complex dose by direct contact | |
|---|---|
| **T4O Concentration** | **100% Mortality time** |
| Negative Control | > 30 minutes |
| 0.5% | 8 minutes |
| 1% | 4 minutes |
| 2% | 3 minutes |
| Commercial towelettes | 15 minutes |
| Cliradex | 3 minutes |

| | |
|---|---|
| T4O: Terpinen-4-ol | |

### Conclusions

A clear dose dependency of the acaricidal effect of the cross-linked hyaluronic Acid/Terpinen-4-ol Complex against DP was highlighted.

### EXAMPLE 6

### In vivo Skin Tolerability of Wipes Soaked with the Composition

An *in vivo* study on healthy volunteers was carried out to evaluate the subjective perception of the use of a wipe soaked with the same concentration (0.5%) of Tea Tree Oil (TTO), Terpinen-4-ol (T4O), and a Complex of cross-linked (urea) hyaluronic acid/Terpinen-4-ol.

The test was performed with the aim of verifying whether the cross-linked hyaluronic acid/terpinen-4-ol complex has comparable or better organoleptic characteristics than TTO or T4O.

For this purpose, 15 healthy volunteers, aged 27 to 65, 9 women and 6 men, were treated with the wipes described above, blindly evaluating four variables: feeling of freshness around the eye area, perceived smell, absence of local irritation, and feel to the touch, using a 5 score scale (5 = very pleasant, 4 = pleasant, 3 = neutral, 2 = unpleasant, 1 = very unpleasant).

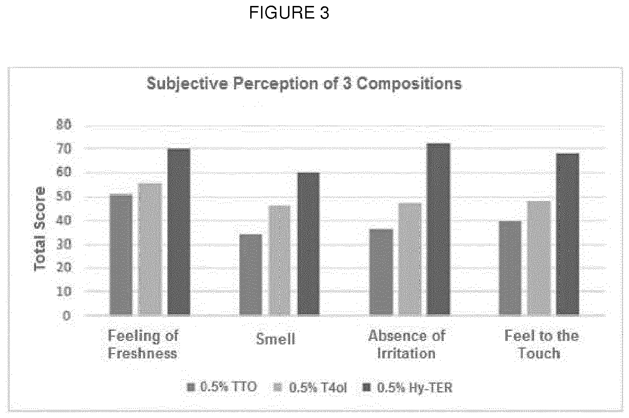

The results are shown in the appended Figure 3.

### Conclusions

The inclusion of Terpinen-4-ol in the cross-linked hyaluronic acid complex has improved the organoleptic characteristics of the product.

## Claims

1. A topical composition comprising
- a component with acaricidal and biocidal activity
- a physiologically acceptable cross-linked polymer, and a dermatologically acceptable carrier,
**characterized in that** the acaricidal and biocidal component is terpinen-4-ol and the cross-linked polymer is hyaluronic acid cross-linked with urea.

2. A composition according to claim 1, wherein the polymer of hyaluronic acid cross-linked with urea has a molecular weight ranging from 100,000 to 20,000,000 dalton, preferably from 2,000,000 to 4,000,000 dalton.

3. A composition according to any one of the claims 1-2, wherein the cross-linked hyaluronic acid with urea and terpinen-4-ol form a complex.

4. A complex for the prevention and/or treatment of ophthalmic and/or dermatological diseases based on hyaluronic acid cross-linked with urea and terpinen-4-ol.

5. A device comprising a solid support wetted with a composition according to any one of the claims 1-3.

6. A device according to claim 5, **characterized in that** said solid support is made of a material selected from cotton wool, non-woven fabric and polyurethane, and it is preferably wetted with a quantity of composition ranging from 0.001 to 0.1 g/cm².

7. Use of a composition according to any one of the claims 1-3, or a complex according to claim 4, or a device according to claim 5 or 6 to cleanse and/or sanitize the skin, the surface of the eye or its appendages.

8. A composition according to any one of the claims 1-3, or complex according to claim 4, for use in the prevention or treatment of ophthalmic diseases.

9. Cosmetic use of a composition according to claim 1 or 3 or complex according to claim 4, in the detersion or hygiene of the periocular area, eyelid or eyelashes.

10. A device according to claim 5 which is a wipe, a gauze, a towelette for application to the ocular and/or periocular area.

11. A composition according to any one of the claims 1-3, or complex according to claim 4, for use in the treatment of blepharitis, acute or chronic conjunctivitis, conjunctivitis with allergic component, or in the treatment of infections of the eye caused by mites, in particular *Demodex, Cheyletiella mite,* or in the treatment of skin diseases, in particular caused by mites, in particular acne or rosacea.

## Patentansprüche

1. Topische Zusammensetzung, umfassend
- eine Komponente mit akarizider und/oder biozider Wirkung,
- ein physiologisch akzeptables vernetztes Polymer und einen dermatologisch akzeptablen Träger,
**dadurch gekennzeichnet, dass** die akarizide und biozide Komponente Terpinen-4-ol ist und das vernetzte Polymer Hyaluronsäure ist, die mit Harnstoff vernetzt ist.

2. Zusammensetzung nach Anspruch 1, wobei das Polymer aus mit Harnstoff vernetzter Hyaluronsäure ein Molekulargewicht im Bereich von 100.000 bis 20.000.000 Dalton, vorzugsweise von 2.000.000 bis 4.000.000 Dalton, aufweist.

3. Zusammensetzung nach einem der Ansprüche 1-2, wobei die vernetzte Hyaluronsäure mit Harnstoff und Terpinen-4-ol einen Komplex bildet.

4. Komplex zur Vorbeugung und/oder Behandlung ophthalmischer und/oder dermatologischer Erkrankungen auf der Basis von Hyaluronsäure, die mit Harnstoff und Terpinen-4-ol vernetzt ist.

5. Vorrichtung mit einem festen Träger, der mit einer Zusammensetzung nach einem der Ansprüche 1-3 benetzt ist.

6. Vorrichtung nach Anspruch 5, **dadurch gekennzeichnet, dass** der feste Träger aus einem Material hergestellt ist, das aus Watte, Vliesstoff und Polyurethan ausgewählt ist, und dass er vorzugsweise mit einer Menge der Zusammensetzung im Bereich von 0,001 bis 0,1 g/cm² benetzt ist.

7. Verwendung einer Zusammensetzung nach einem der Ansprüche 1-3 oder eines Komplexes nach Anspruch 4 oder einer Vorrichtung nach Anspruch 5 oder 6 zur Reinigung und/oder Desinfektion der Haut, der Oberfläche des Auges oder seiner Anhängsel.

8. Zusammensetzung nach einem der Ansprüche 1-3 oder ein Komplex nach Anspruch 4 zur Verwendung bei der Vorbeugung oder Behandlung von Augenkrankheiten.

9. Kosmetische Verwendung einer Zusammensetzung nach Anspruch 1 oder 3 oder eines Komplexes nach Anspruch 4 zur Detersion oder Hygiene des periocularen Bereichs, des Augenlids oder der Wimpern.

10. Vorrichtung nach Anspruch 5, die ein Wischtuch, eine Gaze, ein Handtuch zur Anwendung am Augen- und/oder Augengegendbereich ist.

11. Zusammensetzung zur Verwendung nach einem der Ansprüche 1 - 3 oder Komplex nach Anspruch 4 zur Verwendung bei der Behandlung von Blepharitis, akuter oder chronischer Bindehautentzündung, Bindehautentzündung mit allergischer Komponente oder bei der Behandlung von Infektionen des Auges, die durch Milben, insbesondere *Demodex-, Cheyletiella-Milben,* verursacht werden, oder bei der Behandlung von Hautkrankheiten, die insbesondere durch Milben, insbesondere Akne oder Rosazea, verursacht werden.

## Revendications

1. Une composition topique comprenant
- un composant à activité acaricide et biocide
- un polymère réticulé physiologiquement acceptable, et un véhicule dermatologiquement acceptable,
**caractérisée en ce que** le composant acaricide et biocide est du terpinène-4-ol et le polymère réticulé est de l'acide hyaluronique réticulé avec de l'urée.

2. Une composition selon la revendication 1, dans laquelle le polymère d'acide hyaluronique réticulé avec de l'urée a un poids moléculaire allant de 100 000 à 20 000 000 daltons, préférablement de 2 000 000 à 4 000 000 daltons.

3. Une composition selon l'une quelconque des revendications 1 à 2, dans laquelle l'acide hyaluronique réticulé avec de l'urée et le terpinène-4-ol forment un complexe.

4. Un complexe pour la prévention et/ou le traitement de maladies ophtalmiques et/ou dermatologiques à base d'acide hyaluronique réticulé avec de l'urée et de terpinène-4-ol.

5. Un dispositif comprenant un support solide humidifié avec une composition selon l'une quelconque des revendications 1 à 3.

6. Un dispositif selon la revendication 5, **caractérisé en ce que** ledit support solide est constitué d'un matériau sélectionné parmi de la laine de coton, de l'étoffe non tissée et du polyuréthane, et il est préférablement humidifié avec une quantité de composition allant de 0,001 à 0,1 g/cm².

7. Utilisation d'une composition selon l'une quelconque des revendications 1 à 3, ou un complexe selon la revendication 4, ou un dispositif selon la revendication 5 ou la revendication 6 pour nettoyer et/ou assainir la peau, la surface de l'œil ou ses appendices.

8. Une composition selon l'une quelconque des revendications 1 à 3, ou complexe selon la revendication 4, pour une utilisation dans la prévention ou le traitement de maladies ophtalmiques.

9. Utilisation cosmétique d'une composition selon la revendication 1 ou la revendication 3 ou complexe selon la revendication 4, dans la détersion ou l'hygiène de la zone périoculaire, de la paupière ou des cils.

10. Un dispositif selon la revendication 5 qui est une lingette, une gaze, une chiffonnette pour application sur la zone oculaire et/ou périoculaire.

11. Une composition selon l'une quelconque des revendications 1 à 3, ou complexe selon la revendication 4, pour une utilisation dans le traitement de la blépharite, de la conjonctivite aiguë ou chronique, de la conjonctivite avec un composant allergique, ou dans le traitement d'infections de l'œil provoquées par des acariens, en particulier des démodex, des cheylétielles, ou dans le traitement de maladies de la peau, en particulier provoquées par des acariens, en particulier l'acné ou la rosacée.
